# EUROPEAN PATENT SPECIFICATION

(11) **EP 3 019 194 B1**
(45) Date of publication and mention of the grant of the patent: **17.10.2018**
(21) Application number: 14755175.8
(22) Date of filing: 08.07.2014
(51) Int. Cl.: A61K 41/00, C07K 14/00, C12N 9/99

(54) **PRODUCTION OF SELENOPROTEINS (SELPROT)**
HERSTELLUNG VON SELENOPROTEINEN (SELPROT)
PRODUCTION DE SÉLÉNOPROTÉINES (SELPROT)

(30) Priority: 09.07.2013 LT 2013069
(43) Date of publication of application: 18.05.2016
(73) Proprietor: Vilnius University, 01513 Vilnius (LT)
(72) Inventor: KLIMASAUSKAS, Saulius, LT-10220 Vilnius (LT); RAKAUSKAITE , Rasa, LT-49497 Kaunas (LT); MASEVICIUS, Viktoras, LT-03225 Vilnius (LT)
(74) Representative: Petniunaite, Jurga
(86) International application number: PCT/LT2014/000009
(87) International publication number: WO 2015/005756

(56) References cited:
- WANG LEI ET AL: "Expanding the genetic code", ANGEWANDTE CHEMIE INTERNATIONAL EDITION, WILEY - V C H VERLAG GMBH & CO. KGAA, DE, vol. 44, no. 1, 17 December 2004 (2004-12-17), pages 34-66, XP002548374, ISSN: 1433-7851, DOI: 10.1002/ANIE.200460627 [retrieved on 2004-12-15]
- YOUNG TRAVIS S ET AL: "Expanding the Genetic Repertoire of the Methylotrophic Yeast Pichia pastoris", BIOCHEMISTRY, AMERICAN CHEMICAL SOCIETY, US, vol. 48, no. 12, 31 March 2009 (2009-03-31), pages 2643-2653, XP002631536, ISSN: 0006-2960, DOI: 10.1021/BI802178K [retrieved on 2009-03-05]
- WU NING ET AL: "A genetically encoded photocaged amino acid", JOURNAL OF THE AMERICAN CHEMICAL SOCIETY, AMERICAN CHEMICAL SOCIETY, vol. 126, no. 44, 10 November 2004 (2004-11-10), pages 14306-14307, XP002459777, ISSN: 0002-7863, DOI: 10.1021/JA040175Z [retrieved on 2004-10-19]
- QING SHAO ET AL: "Photoactive molecules for applications in molecular imaging and cell biology", CHEMICAL SOCIETY REVIEWS, vol. 39, no. 8, 1 January 2010 (2010-01-01), page 2835, XP055157312, ISSN: 0306-0012, DOI: 10.1039/b915574k
- D. GFELLER ET AL: "SwissSidechain: a molecular and structural database of non-natural sidechains", NUCLEIC ACIDS RESEARCH, vol. 41, no. D1, 26 October 2012 (2012-10-26), pages D327-D332, XP055157331, ISSN: 0305-1048, DOI: 10.1093/nar/gks991
- RASA RAKAUSKAITE ET AL: "Production of selenoproteins in yeast via genetically encoded incorporation of a photocaged selenocysteine", NEW BIOTECHNOLOGY, vol. 31, 1 July 2014 (2014-07-01), page S202, XP055157074, ISSN: 1871-6784, DOI: 10.1016/j.nbt.2014.05.969

## Description

### Field of the Invention

The present invention relates to targeted incorporation of rare and unnatural amino acids into proteins, in particular to the use of in vivo technology utilizing UAG codon suppression by genetically encoded unnatural amino acids for recombinant protein synthesis.

### Background of the Invention

Engineering and in-cell production of recombinant proteins is widely exploited for structural and functional studies and for numerous practical applications in medicine and industry. However, designing and producing a protein with a desired enzymatic activity or biological function is often a challenging task (Brustad, E.M. and Arnold, F.H. (2011) Curr. Opin. Chem. Biol. 15(2), 201-210). Incorporation of selenocysteine (Sec), the 21st amino acid, into engineered proteins is one of the ways to bring in new properties. Sec is a cysteine (Cys) analog carrying a selenium atom instead of sulfur, which provides unique physicochemical characteristics to Sec. When compared to the thiol group of Cys, the selenol group of Sec has a higher nucleophilicity, lower pKa, and a lower redox potential. Due to its pronounced chemical reactivity, Sec is capable of changing structural and enzymatic qualities of a protein. For example, engineering selenocysteine residues into the catalytic centre offers an attractive means to expand enzyme's naturally evolved catalytic capacity (Huang, X. et al. (2011) Chem. Soc. Rev. 40, 1171-1184). Alternatively, the unique chemical reactivity of Sec towards electrophiles and oxidation reagents makes it useful for selective conjugation, labeling or creation of dimerization sites in a protein (Heras, I.L. et al. (2011) Anal. Bioanal. Chem. 400(6), 1717-27; Arnér, E. (2010) Exp. Cell. Res. 316(8), 1296-303; Johansson, L. et al (2005) Biochim. Biophys. Acta. 1726(1), 1-13). In addition, incorporation of selenocysteine carrying valuable isotopes of Se is useful for facilitation of phase determination by multi-wavelength anomalous diffraction in X-ray crystallography of proteins, positron emission tomography PET (⁷³Se), specific radiolabeling (⁷⁵Se) and high-resolution NMR studies (⁷⁷Se) of proteins (Johansson, L. et al (2005) Biochim. Biophys. Acta. 1726(1), 1-13). Yet another area is efficient production of natural recombinant selenoproteins in bacterial (Arnér, E.S.J. (2002) Met. Enzymol. 347, 226-235) or eukaryotic cells (Liu, H. et al. (2012) Protein Expr Purif. 84(1), 59-63.

Despite its high technological potential, incorporation of Sec into recombinant proteins is far from trivial. A number of methods have been proposed for making artificial selenoproteins, however, none of them so far enables a precise insertion of Sec with a good yield (Johansson, L. et al (2005) Biochim. Biophys. Acta. 1726(1), 1-13). For example, protein ligation approaches are limited by the solid base synthesis which generally allows making peptides no longer than 50 amino acids. If Sec has to be incorporated beyond the 50^{th} position, ligation of 3 peptide fragments is needed. Since peptide ligation requires an N-terminal Cys residue, this approach unavoidably creates an additional Cys mutation in the protein sequence (Muir, T. W. (2003) Annu. Rev. Biochem. 72, 249-289). Chemical conversion of serine (Ser) residues to Sec targets all Ser residues and therefore is not suitable for making single Sec substitutions in most proteins (Wu, Z.-P., Hilvert, D. (1989) J. Am. Chem. Soc. 111, 4513-4514). A similar lack of specificity arises when a protein containing several Cys residues is expressed in a Cys auxotrofic strain with Sec supplied in the growth medium (Muller, S. et al. (1994) Biochem. 33, 3404-3412). Expression of recombinant proteins in bacteria by employing native UGA codon suppression is also technically limited. Specifically, bacterial selenoprotein genes carry a hairpin-like selenocystein insertion sequence (SECIS) inside the open reading frame and adjacent to the UGA codon. Not only the SECIS structure but also 4 conserved nucleotides within it are absolutely required to direct the incorporation of Sec into the peptide chain during translation (Yoshizawa, S., Böck, A. (2009) Biochim. et Biophys. Acta. 1790, 1404-1414). Therefore, it is practically impossible to engineer a SECIS structure into a recombinant mRNA sequence without making additional changes in the protein sequence. Mammalian expression systems, in which SECIS is positioned in the 3'UTR, are suitable for making targeted Sec incorporation without mandatory alterations of the protein sequence, but cannot provide good protein yields at a comparable cost (Berry, M.J. et al. (1993) EMBO J., 12 (8), 3315-3322). The same is true about *in vitro* protein expression systems which require expensive components and labor-intensive chemical charging of tRNA with Sec (Kang, T.J., Suga, H. (2008) Biochem. Cell Biol. 86, 92-99).

An alternative approach for targeted incorporation of certain unnatural amino acids is based on *in vivo* incorporation of genetically encoded unnatural amino acids at a UAG nonsense codon. This technology allowed incorporation of over 70 different unnatural amino acids into protein structures with high specificity (Liu, C.C. and Schultz, P.G. (2010) Annu. Rev. Biochem. 79, 413-444), however, it has not been shown to be suitable for producing Sec insertions/substitutions. Although successful incorporation of phenylselenocysteine into histone H3 protein was achieved (Guo, J. et al. (2008) Angew. Chem. Int. Ed. Engl. 47(34), 6399-6401), but a suitable method for deprotection of the incorporated residue to Sec was not proposed (Clark, K.M. et al. (2009) Methods Enzymol. 462, 97-115). This approach was also used for the incorporation of photocaged amino acids such as DMNB-Ser, which can be readily converted to Ser *in situ* by photolysis (Lemke, E.A. et al. (2007) Nat. Chem. Biol. 3(12), 769-772). The yeast cells are co-transformed with the pescTrpLeuRSBH5T252A plasmid, which encodes for an orthogonal UAG nonsense codon suppressor tRNA_{CUA} (O-tRNA) and an orthogonal aminoacyl tRNA synthetase (O-RS) that is capable of charging the O-tRNA with the unnatural amino acid DMNB-Ser (and apparently DMNB-Cys, Young, T.S. et al. (2009) Biochemistry. 48(12), 2643-2653). However, the amounts of such recombinant proteins obtained from the described yeast cultures were quite low (Lemke, E.A. et al. (2007) Nat. Chem. Biol. 3(12), 769-772; Chin, J.W. et al. (2003) Science. 301(5635), 964-967). The yield of recombinant proteins was improved when expression of O-tRNA was optimized by fusing its gene to the *SNR*52 promoter (pSNR-LeuRS plasmid) and expressing the protein in a yeast *UPF*1 deletion strain (LWUPF1Δ), which stabilizes nonsense containing mRNAs (Wang, Q. and Wang, L. (2008) J. Am. Chem. Soc. 130(19), 6066-6067). However, the described system was not used for expression of DMNB-Ser or DMNB-Cys containing proteins.

So far none of the existing technologies proved to serve as a general method for high yield production of recombinant proteins that contain a Sec residue in a predefined position.

### Summary of the Invention

First, the current invention solves the above problem by using a UAG codon engineered at any defined position of mRNA, which is decoded in yeast cells by the O-tRNA charged with the unnatural amino acid, the photocaged selenocysteine. The protective group such as 4,5-dimethoxy-2-nitrobenzyl (DMNB) is then removed from the protein by illumination with UV light leaving Sec in place. This strategy, in principle, can be applied to make any desired Sec incorporation in any protein. Moreover, in live cells, synthesis of a photocaged selenoprotein will permit optical control over biological processes carried out by the protein. As a result, this method offers a new tool for protein engineering and specifically selenology.

Second, the current invention provides a new photocaged selenocysteine compound of formula I and a synthesis scheme for obtaining the same.

### Brief Description of the Figures

Figure 1 shows synthetic route to production of DMNB-Cys.
Figure 2 shows synthetic route to production of DMNB-Sec.
Figure 3 illustrates DMNB-Sec-dependent production of functional EGFP protein in yeast S. *cerevisiae* cells.
Figure 4 illustrates the results of the removal of the photocaging DMNB group from cysteine and selenocysteine residues after exposing the EGFP protein to UV light (340-390 nm).
Figure 5 illustrates the results of the formation of the EGFP protein dimers after protein exposure to uv and reduction of the dimers by DTT treatment.
Figure 6 demonstrates that photodecaging, dimerisation and reductive monomerisation all occur under mild conditions preserving the functionally (fluorescence) of the EGFP protein.

### Detailed Description of the Invention

Unless defined herein, all technical and scientific terms used herein have the same meaning as commonly understood by one of ordinary skill in the art to which the invention pertains.

The term "orthogonal" refers to a molecule that functions with endogenous components of a cell with reduced efficiency as compared to a corresponding molecule that is endogenous to the cell or translation system, or that fails to function with endogenous components of the cell. In the context of tRNAs and aminoacyl-tRNA synthetases, orthogonal refers to an inability or reduced efficiency of an orthogonal tRNA to function with an endogenous tRNA synthetase compared to an endogenous tRNA to function with the endogenous tRNA synthetase, or of an orthogonal aminoacyl-tRNA synthetase to function with an endogenous tRNA compared to an endogenous tRNA synthetase to function with the endogenous tRNA. The orthogonal molecule lacks a functionally normal endogenous complementary molecule in the cell. The term "selector codon" refers to codons recognized by the O-tRNA in the translation process and not recognized by an endogenous tRNA. Selector codons can include, e.g., nonsense codons, such as UAG, UAA, UGA. The term "translation system" refers to the components that incorporate an amino acid into a growing polypeptide chain; these components can include, for example, ribosomes, tRNAs, synthetases, mRNA and the like. The O-tRNA and the O-RS can be added to or be a part of an in vitro or in vivo translation system. The compositions made by the methods of the invention optionally are in a cell. The unnatural amino acid that is added to the system can be a synthetic amino acid, such as a photocaged selenocysteine, which can be exogenously added to the growth medium.

The current invention relates to a method for producing in a translation system a protein comprising an unnatural amino acid at one or more selected positions, the method comprising:
(a) providing a translation system comprising:
   (i) an unnatural amino acid that is photocaged selenocysteine;
   (ii) an orthogonal aminoacyl-tRNA synthetase (O-RS);
   (iii) an orthogonal tRNA (O-tRNA), wherein said O-RS preferentially aminoacylates said O-tRNA with said photocaged selenocysteine; and,
   (iv) a nucleic acid encoding said protein, wherein said nucleic acid comprises at least one selector codon that is recognized by said O-tRNA; and,
(b) incorporating said unnatural amino acid at said selected position in said protein during translation of said protein in response to said selector codon, thereby producing said protein comprising said unnatural amino acid at the selected position.

The method may further comprise step (c) removing the photocaging group by exposing translation system or purified protein solution to UV light.
The host cell that is used is preferably a bacterial, yeast or mammalian host cell; more preferably it is a yeast cell. The yeast cell is preferably *Saccharomyces cerevisiae.*
The invention further relates to a photocaged selenocysteine of the formula I: 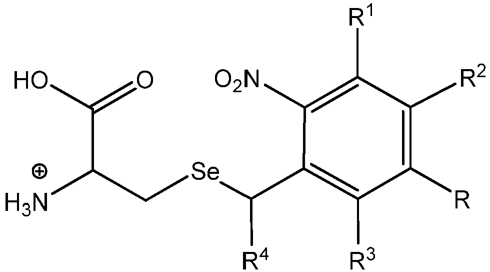 wherein
R is H, alkyl or alkoxy, carboxymethoxy group,
R¹ is H, alkyl, aryl, alkoxy, hydroxy, or a halide group.
R² and R³ each independently represent H, alkyl, aryl, alkoxy, hydroxy group, CO₂R⁵ or halide; wherein when R and R² or R and R³ is alkoxy, R² or R³ may form a five, six or seven membered cyclic structure in combination with R,
R⁴ is H, methyl, alkyl, CH₂OR⁵ or CO₂R⁵
R⁵ is H or alkyl.
The photocaged selenocysteine is preferably 4,5-dimethoxy-2-nitrobenzylselenocysteine (DMNB-Sec).
The invention further relates to a process for producing a photocaged selenocysteine of formula I, which comprises the steps of: reducing of N,N'-blocked selenocystine and reacting with a compound selected from a corresponding substituted (at positions 1,3,4,5 and/or alpha) 2-nitrobenzyl halide, sulfonate, phosphate or carboxylate, followed by de-protection of amino groups. The selenocystine may be blocked with a group selected from butoxycarbonyl, carboxybenzyl, allyloxycarbonyl, metoxy or ethoxycarbonyl.
The photocaged selenocysteine of formula I may be further used in the method of invention.

The present invention for site-specific Sec incorporation is based on UAG codon suppression technology used to incorporate some other unnatural amino acids into proteins. This method employs yeast cells transformed with plasmids, carrying an engineered UAG nonsense codon at the desired location inside a gene of interest.
The system comprises the following major components:
1. the *UPF*1-deficient LWUPF1Δ yeast strain;
2. plasmid pRR6;
3. synthetic photocaged amino acid DMNB-Sec
4. plasmid carrying an engineered UAG nonsense codon at the desired location inside a gene of interest (exemplified by pGFP-TAG);

The pRR6 plasmid was constructed by replacing the LeuRS gene in the pSNR-LeuRS plasmid (Wang, Q. and Wang, L. (2008) J. Am. Chem. Soc. 130(19), 6066-6067) with the mutated DMNB-Ser specific LeuRSBH5T252A gene from the pescTrpLeuRSBH5T252A plasmid (Lemke, E.A. et al. (2007) Nat. Chem. Biol. 3(12), 769-772) by genetic engineering. The nucleotide sequence representing LeuRSBH5T252A gene is provided as SEQ ID NO: 1.

atgcaagagcaataccgcccggaagagatagaatccaaagtacagcttcattgggatgagaagcgcacatttgaagtaaccgaagacgag
agcaaagagaagtattactgcctgtctgggcagccctatccttctggtcgactacacatgggccacgtacgtaactacaccatcggtgacgt
gatcgcccgctaccagcgtatgctgggcaaaaacgtcctgcagccgatcggctgggacgcgtttggtctgcctgcggaaggcgcggcgg
tgaaaaacaacaccgctccggcaccgtggacgtacgacaacatcgcgtatatgaaaaaccagctcaaaatgctgggctttggttatgactg
gagccgcgagctggcaacctgtacgccggaatactaccgttgggaacagaaattcttcaccgagctgtataaaaaaggcctggtatataag
aagacttctgcggtcaactggtgtccgaacgaccagaccgtactggcgaacgaacaagttatcgacggctgctgctggcgctgcgatacc
aaagttgaacgtaaagagatcccgcagtggtttatcaaaatcactgcttacgctgacgagctgctcaacgatctggataaactggatcactgg
ccagacaccgttaaaaccatgcagcgtaactggatcggtcgttccgaaggcgtggagatcaccttcaacgttaacgactatgacaacacgc
tgaccgtttacactacccgcccggacgcgtttatgggttgtacctacctggcggtagctgcgggtcatccgctggcgcagaaagcggcgga
aaataatcctgaactggcggcctttattgacgaatgccgtaacaccaaagttgccgaagctgaaatggcgacgatggagaaaaaaggcgtc
gatactggctttaaagcggttcacccattaacgggcgaagaaattcccgtttgggcagcaaacttcgtattgatggagtacggcacgggcgc
agttatggcggtaccggggcacgaccagcgcgactacgagtttgcctctaaatacggcctgaacatcaaaccggttatcctggcagctgac
ggctctgagccagatctttctcagcaagccctgactgaaaaaggcgtgctgttcaactctggcgagttcaacggtcttgaccatgaagcggc
cttcaacgccatcgccgataaactgactgcgatgggcgttggcgagcgtaaagtgaactaccgcctgcgcgactggggtgtttcccgtcag
cgttactggggcgcgccgattccgatggtgactctagaagacggtaccgtaatgccgaccccggacgaccagctgccggtgatcctgccg
gaagatgtggtaatggacggcattaccagcccgattaaagcagatccggagtgggcgaaaactaccgttaacggtatgccagcactgcgt
gaaaccgacactttcgacacctttatggagtcctcctggctgtatgcgcgctacacttgcccgcagtacaaagaaggtatgctggattccgaa
gcggctaactactggctgccggtggatatcggtattggtggtattgaacacgccattatgtttctgctctacttccgcttcttccacaaactgatg
cgtgatgcaggcatggtgaactctgacgaaccagcgaaacagttgctgtgtcagggtatggtgctggcagatgccttctactatgttggcga
aaacggcgaacgtaactgggtttccccggttgatgctatcgttgaacgtgacgagaaaggccgtatcgtgaaagcgaaagatgcggcagg
ccatgaactggtttataccggcatgagcaaaatgtccaagtcgaagaacaacggtatcgacccgcaggtgatggttgaacgttacggcgcg
gacaccgttcgtctgtttatgatgtttgcttctccggctgatatgactctcgaatggcaggaatccggtgtggaaggggctaaccgcttcctga

aacgtgtctggaaactggtttacgagcacacagcaaaaggtgatgttgcggcactgaacgttgatgcgctgactgaaaatcagaaagcgct
gcgtcgcgatgtgcataaaacgatcgctaaagtgaccgatgatatcggccgtcgtcagaccttcaacaccgcaattgcggcgattatggag
ctgatgaacaaactggcgaaagcaccaaccgatggcgagcaggatcgcgctctgatgcaggaagcactgctggccgttgtccgtatgctt
aacccgttcaccccgcacatctgcttcacgctgtggcaggaactgaaaggcgaaggcgatatcgacaacgcgccgtggccggttgctgac
gaaaaagcgatggtggaagactccacgctggtcgtggtgcaggttaacggtaaagtccgtgccaaaatcaccgttccggtggacgcaacg
gaagaacaggttcgcgaacgtgctggccaggaacatctggtagcaaaatatcttgatggcgttactgtacgtaaagtgatttacgtaccaggt
aaactcctcaatctggtcgttggctaa

The photocaged selenocysteine, DMNB-Sec, was chemically synthesized from selenocystine as a trifluoroacetate salt using a newly developed procedure. The same procedure may be used to synthesize other photocaged selenocysteines of the formula I: 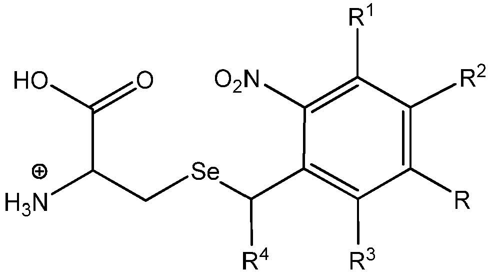 Wherein
R is H, alkyl or alkoxy, carboxymethoxy group,
R¹ is H, alkyl, aryl, alkoxy, hydroxy, or a halide group.
R² and R³ each independently represent H, alkyl, aryl, alkoxy, hydroxy group, CO₂R⁵ or halide; wherein when R and R² or R³ is alkoxy, R² or R³ may form a five, six or seven membered cyclic structure in combination with R,
R⁴ is H, methyl, alkyl, CH₂OR⁵ or CO₂R⁵
R⁵ is H or alkyl.

As an example, we used plasmid pGFP-TAG (Wang, Q. and Wang, L. (2008) J. Am. Chem. Soc. 130(19), 6066-6067), which encodes for the EGFP protein with a UAG mutation at non-essential position Tyr39.

For protein production, the LWUPF1Δ cells were simultaneously transformed with the pRR6 and pGFP-TAG plasmids and were grown in medium containing 4 mM DMNB-Sec. The full length active protein is formed only when the unnatural amino acid is inserted to its sequence after decoding the UAG codon. The 6-His tagged EGFP protein carrying the unnatural amino acid DMNB-Sec replacing Tyr39 was purified from yeast cells using Immobilized metal affinity chromatography.
The identity of the isolated EGFP protein carrying DMNB-Sec was confirmed by mass spectrometry. The yield of the seleno-EGFP protein was 1 mg/L.
The disclosed approach uses a photocaging DMNB group to protect Sec, such that the reactive selenohydryl (or selenide) group will be protected from chemical reactions with the biopolymers, metabolites and other molecules (including reactive oxygen species) present in cellular environment. After protein synthesis is complete, the photocaging group can be removed by illuminating the producing cells or isolated protein solution *in vitro* with UV light.

Previously, it was shown that the O-RS optimized for DNMB-Ser can also accept DNMB-Cys (Young, T.S. et al. (2009) Biochemistry. 48(12), 2643-2653). We surprisingly found that this aaRS can also accept DMNB-Sec. Although the DMNB-protected Sec differs by only one atom from DMNB-Ser and DMNB-Cys, it was not directly obvious that such a replacement in the unnatural amino acid would be well tolerated by the mutated orthogonal LeuRS. One of the reasons is a noticeably larger van der Waals radius of selenium as compared to sulfur and oxygen, and the larger amino acid DMNB-Sec is less likely to fit in the pocket of an enzyme. Secondly, a much higher chemical reactivity of selenium towards electrophiles and oxidants might lead to fast decay of DMNB-Sec in the growth medium or inside the yeast cells. Third, the toxicity of 4 mM DMNB-Sec for yeast cells was not previously examined, whereas, some Se compounds are known to be toxic for yeast at much lower concentrations (SeMet is toxic at 0.25 mM, Kitajima, T. et al. (2012) J. Biol. Chem. 287(13), 10032-8). Fourth, although the thiol chemistry in general has certain similarities with selenium chemistry, we found that a synthetic route established for production of DMNB-Cys from cystine was unsuitable and required major changes to be efficient for the synthesis of DMNB-Sec from selenocystine. Finally, photochemical removal of the caging DMNB group from Sec or other Se compounds was not previously described and it was not clear if the reaction would proceed in a similar fashion as observed for Ser (Lemke, E.A. et al. (2007) Nat. Chem. Biol. 3(12), 769-772) or Cys (Rathert, P. et al., (2007) Chembiochem. 8(2), 202-207.).

The described approach can be used for production of any recombinant selenoprotein in yeast. The list of known selenoproteins is provided in the following resource: http://www.selenodb.org/

The described approach can be used for production of any other artificially modified proteins in which one or several Sec residues are inserted in desired positions. Inserted Sec residues can be used for targeted labeling or further chemical modification of the protein.
Sec residues are preferentially labelled with the same reagents used for Cys modification, but the reaction can be selectively performed at Sec at a slightly acid pH (pH<7).
Inserted Sec residues can be used for targeted and light-controlled dimerization of functional proteins as exemplified for EGFP.

The described approach can be used for production of any artificially modified enzymes in which one or several Sec residues are inserted by replacing catalytic Cys or Ser residues in an enzyme. Preferential classes of such enzymes are thiol oxidoreductases (EC 1.8.3.2), protein-tyrosine phosphatases (EC 3.1.3.48), Cys peptidases (EC 3.4.22) (The MEROPS online database: //merops.sanger.ac.uk/cgi-bin/family_index?type=P#C), one carbon pyrimidine-5 transferases such as deoxycytidylate 5-hydroxymethyltransferases (EC 2.1.2.8), thymidylate synthetases (EC 2.1.1.45), and a large variety of DNA cytosine-5 methyltransferases (EC 2.1.1.37), RNA cytosine-5 methyltransferases (listed in http://modomics.genesilico.pl/reactions/C:m5C/) and RNA uracil-5 methyltransferases (listed in http://modomics.genesilico.pl/reactions/U:m5U/) (Bujnicki J. et al. (2004) Nucleic Acids Res. 32(8), 2453-63). DNA cytosine-5 methyltransferases (exemplified by M.HhaI, M.SssI, M.HpaII, M.AluI, M2.Eco31I) and their engineered variants have also been shown to catalyze sequence-specific non-canonical reactions such as the transfer of extended groups from synthetic cofactor analogues (Lukinavičius G. et al. (2012) Nucleic Acids Res., 40(22), 11594-11602; US2013130922A1), addition and removal of formaldehyde at the 5-position of a target cytosine residue (Liutkevičiutė Z. et al., (2009) Nat. Chem. Biol. 5, 400-402), and addition of aliphatic thiols or selenols to 5-hydroxymethylcytosine residues in DNA (Liutkevičiutė Z. et al., (2011) Angew. Chem. Int. Ed., 50, 2090-2093). These novel reactions can be used for analysis of epigenetic modifications in genomic DNA which typically occur at CpG dinucleotide sequences. Replacement of the catalytic Cys with Sec may enhance the catalytic power of these enzymes in these practically important non-canonical reactions.

### EXAMPLES

### Example 1. Synthesis of photocaged amino acids

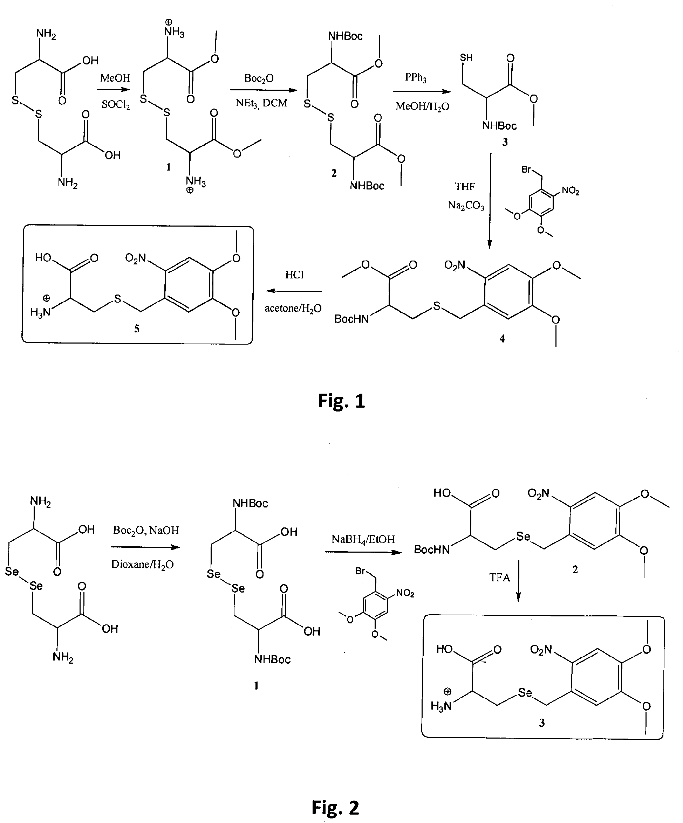

### Synthesis of DMNB-Cys (Figure 1.)

### Cystine dimethyl ester 1

Suspension of cystine (5 g, 20.8 mmol) in MeOH (100 ml) was cooled to -70°C and thionyl chloride (3.75 ml, 19.24 mmol) was added to reaction vessel. Reaction mixture was left until room temperature is reached, then refluxed for 6 hours. Methanol evaporated under reduced pressure and residue dissolved in methanol, after addition of diethyl ether product precipitates as white crystals. Procedure gave 6.69g (94%) of target product as hydrochloride salt.

### N,N'-bis(tert-butoxycarbonyl)cystine dimethyl ester 2

Reaction was performed according procedure described in literature (Busnel et al., (2005) Bioorg. Med. Chem. 13(7), 2373-80).

### N-(tert-butoxycarbonyl)cysteine methyl ester 3

Reaction was performed according procedure described in literature (Busnel et al., (2005)).

### N-(tert-butoxycarbonyl)-S-(4,5-dimethoxy-2-nitrobenzyl)cysteine methyl ester 4

Suspension of *N*-(*tert*-butoxycarbonyl)cysteine methyl ester (1.94 g, 8.25 mmol), 4,5-dimethoxy-2-nitrobenzylbromide (2.40 g, 8.73 mmol) and sodium carbonate (1.51 g, 14.25 mmol) in THF (50 ml) refluxed for 60 hours, then quenched with water (50 ml). THF was evaporated under reduced pressure and product extracted with DCM. DCM evaporated, residue purified by column chromatography (C₆H₆:CHCl₃ 1:1, R_{f} = 0.23). Described procedure yielded 2.16 g (61%) of target product.
¹H NMR (400 MHz, CDCl₃): δ = 1.47 (9H, s, 3×CH₃), 2.91 (1H, dd *J* = 14, 5.6Hz, S*CH_{A}*H_{B}), 2.98 (1H, dd *J* = 14, 5.6Hz, SCH_{A}*H_{B}*), 3.78 (3H, s, OCH₃), 3.97 (3H, s, ArOCH₃), 4.03 (3H, s, ArOCH₃), 4.09 (1H, d *J* = 13.6Hz, BnC*H_{A}*H_{B}S), 4.17 (1H, d *J* = 13.6Hz, BnC*H*_{A}H*_{B}*S), 4.56 (1H, brm, CH), 5.35 (1H, d *J* = 7.6Hz, NH), 6.94 (1H, s, ArH), 7.69 (1H, s, ArH). ¹³C NMR (100 Mz, CDCl₃): δ = 28.3, 34.4, 34.5, 52.7, 53.4, 56.4, 56.5, 80.3, 108.8, 113.4, 128.7, 140.5, 148.0, 153.0, 155.2, 174.4. Anal. calcd. for C₁₈H₂₆N₂O₈S: C, 50.22; H, 6.09; N, 6.51; S, 7.45. Found: C, 50.39; H, 6.25; N, 6.53; S, 7.22. HRMS: m/z [M+Na]⁺ calcd. for C₁₈H₂₆N₂O₈S: 453.1302; Found: 453.1310.

### S-(4,5-dimethoxy-2-nitrobenzyl)cysteine hydrochloride 5

To suspension of *N*-(*tert*-butoxycarbonyl)-*S*-(4,5-dimethoxy-2-nitrobenzyl)cysteine methyl ester (0.95 g, 2.21 mmol) in mixture of acetone (25 ml) and water (25 ml) conc. hydrochloric acid (0,92 ml, 11.36 mmol) was added. Reaction mixture was stirred for 3 days at 60°C (sand bath) temperature, solvents evaporated under reduced pressure, residue washed with acetone. Described procedure yielded 0.74 g (95%) of target product.
¹H NMR (400 MHz, DMSO-D₆): δ = 2.95 (1H, dd *J* = 14.6, 6.4Hz, SC*H_{A}*H_{B}), 3.01 (1H, dd *J* = 14.6,4.8, SCH_{A}*H_{B}*, 3.86 (3H, s, OCH₃), 3.93 (3H, s, OCH₃), 4.12 (1H, d *J* = 13.6 Hz, BnC*H_{A}*H_{B}S), 4.15-4.25 (2H, m, BnCH_{A}*H_{B}*S + CH), 7.35 (1H, s, ArH), 7.70 (1H, s, ArH), 8.62 (3H, brs, NH₃), 14.04 (1H, brs, OH). ¹³C NMR (100 Mz, CDCl₃): δ = 31.3, 33.7, 52.3, 56.5, 57.0, 109.4, 114.9, 129.3, 140.1, 148.0, 153.2, 170.0. HRMS: m/z [M+H]⁺ calcd. for C₁₂H₁₆N₂O₆S: 317.0802; Found: 317.0805.

### Synthesis of DMNB-Sec (Figure 2.)

### N,N'-bis(tert-butoxycarbonyl)selenocystine 1

Solution of selenocystine (0.625 g, 1.88 mmol) in 3.8 ml of alkaline water (1M NaOH) was combined with solution of di-*tert*-butyldicarbonate (1.633 g, 7.49 mmol) in 1.8 ml of dioxane and .reaction mixture was stirred for 24 hours at room temperature, concentrated under reduced pressure and washed with ethyl acetate. Water phase was acidified with hydrochloric acid,and product extracted with ethyl acetate. Organic layer washed with brine, dried over sodium sulphate. Ethyl acetate was removed under reduced pressure. Residue was recrystallized form benzene to yield 0.755 g (75.5%) of target product.
¹H NMR (300 MHz, CDCl₃): δ = 1.45 (9H, s, 3×CH₃), 3.21 (1H, dd *J* = 12.6, 4.8Hz, SeC*H_{A}*H_{B}), 3.46 (1H, dd *J* = 12.6, 4.8Hz, SeCH_{A}*H_{B}*), 4.61 (1H, brm, CH), 5.37 (1H, brs, NH).

### N-(tert-butoxycarbonyl)-Se-(4,5-dimethoxy-2-nitrobenzyl)selenocysteine 2

Mixture of *N*,*N*'-bis(*tert*-butoxycarbonyl)selenocystine (0.755 g, 1.41 mmol) and 4,5-dimethoxy-2-nitrobenzylbromide (1.170 g, 4.24 mmol) in ethanol (30 ml) was cooled to 0°C, then sodium borohydride (0.161 g, 4.26 mmol) poured to reaction vessel. Reaction mixture was stirred for 75 minutes at room temperature then quenched with water (70 ml). Ethyl acetate was added and reaction mixture was acidified with hydrochloric acid. Organic layer was separated and dried over sodium sulphate. Solvents evaporated under reduced pressure, residue purified by column chromatography (CHCl₃, R_{f} = 0.26). Described procedure yielded 1.133 g (86.5%) of target product.
¹H NMR (400 MHz, CDCl₃): δ = 1.46 (9H, s, 3×CH₃), 3.03 (1H, dd *J* = 13.6, 4.8Hz, SeC*H_{A}*H_{B}), 3.09 (1H, dd *J* = 13.6, 4.8Hz, SeCH_{A}*H_{B}*), 3.95 (3H, s, OCH₃), 4.01 (3H, s, OCH₃), 4.11 (1H, d *J* = 11.6Hz, BnC*H_{A}*H_{B}Se), 4.21 (1H, d *J* = 11.6Hz, BnCH_{A}*H_{B}*Se), 4.62 (1H, brm, CH), 5.46 (1H, brm, NH), 6.83 (1H, s, ArH), 7.69 (1H, s, ArH). ¹³C NMR (100 Mz, CDCl₃): δ = 25.9 (2 overlapping signals, it's clear from HSQC data), 28.3, 53.6, 56.4, 56.6, 80.7, 108.9, 113.2, 130.6, 139.7, 147.8, 153.2, 155.6, 174.9. Anal. calcd. for C₁₇H₂₄N₂O₈Se: C, 44.07; H, 5.22. Found: C, 44.13; H, 5.53. HRMS: m/z [M+Na]⁺ calcd. for C₁₇H₂₄N₂O₈Se: 487.0591; Found: 487.0589.

### Se-(4,5-dimethoxy-2-nitrobenzyl)selenocysteine trifluoroacetate 3

*N*-(*tert*-butoxycarbonyl)-*Se*-(4,5-dimethoxy-2-nitrobenzyl)selenocysteine (1.13 g, 2.44 mmol) was dissolved in TFA (10 ml) and stirred for 15 min at room temperature. After TFA was evaporated solid residue was washed with DCM, filtered and dried. Crude product was dissolved in methanol, insoluble impurities filtered off. Evaporation of methanol under reduced pressure yielded 0.9 g (77%) of target product.
¹H NMR (300 MHz, DMSO-D₆): δ = 2.95-3.11 (2H, m, SeCH₂), 3.87 (3H, s, OCH₃), 3.92 (3H, s, OCH₃), 4.14 (1H, d *J* = 12 Hz, BnC*H_{A}*H_{B}Se), 4.17-4.25 (2H, m, BnCH_{A}*H_{B}*Se + CH), 7.15 (1H, s, ArH), 7.70 (1H, s, ArH), 8.44 (3H, brs, NH₃). ¹³C NMR (75 Mz, CDCl₃): δ = 23.4, 26.0, 53.1, 56.7, 57.0, 109.5, 114.5, 131.2, 139.8, 148.2, 153.6, 170.4. HRMS: m/z [M+H]⁺ calcd. for C₁₂H₁₆N₂O₆Se: 365.0247; Found: 365.0255.

### Example 2 Targeted incorporation of a photocaged Sec into a protein produced in yeast cells.

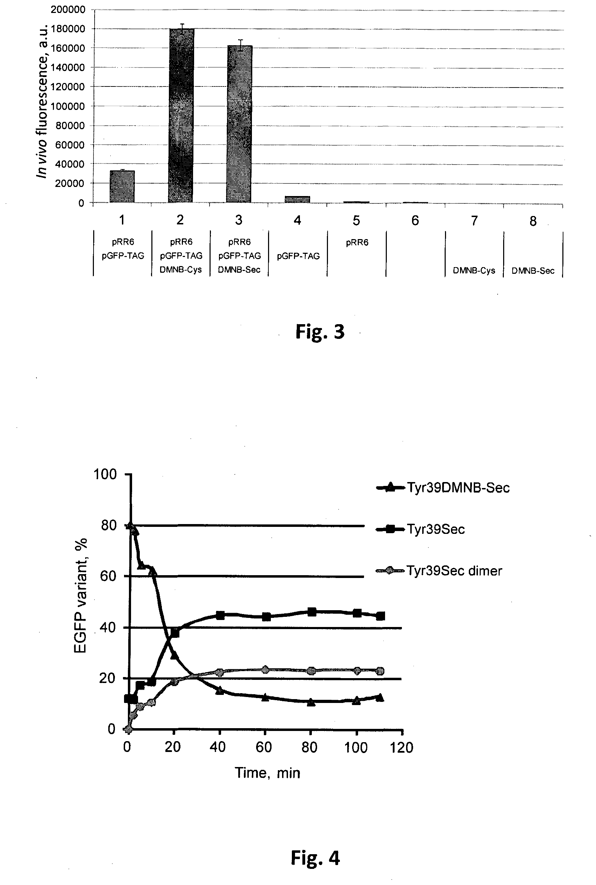

Example 2 demonstrates that the presence of all O-tRNA, O-RS and DMNB-Cys or DMNB-Sec is required for production of a functional protein using the disclosed yeast expression system. Yeast cells were simultaneously transformed with two plasmids (pGFP-TAG and pRR6) as well as single plasmid combinations and selected transformants were grown either without (column 1) or with the unnatural amino acid (columns 2 and 3) in selective liquid medium for 24 h at 30°C. Cells were harvested, washed and resuspended in water. The efficiency of the DMNB-Sec-dependent production of a functional EGFP protein was monitored by measuring EGFP fluorescence of the producing yeast cells (Figure 3). Fluorescence readings were taken from 0.1 ml samples in triplicates using Synergy H4 (BioTek Instruments, Inc.) plate reader. Fluorescence values were corrected by absorbance OD₅₉₅ readings which were taken in parallel for each sample. Plasmid pGFP-TAG encodes an EGFP protein that contains a TAG nonsense codon substitution at functionally permissive Tyr39 position. Plasmid pRR6 encodes a TAG nonsense codon suppressor O-tRNA, and a mutated orthogonal LeuRS. Cells grown without a DMNB-protected amino acid produce mostly truncated nonfluorescent EGFP (column 1). Functional active EGFP protein (fluorescent) is produced after decoding of the TAG codon by a nonsense suppressor O-tRNA charged with unnatural amino acids DMNB-Cys (column 2) or DMNB-Sec (column 3).
Column 1. EGFP fluorescence above the background level in cells grown without unnatural amino acids shows low level production of full length EGFP protein likely due to residual charging of O-tRNA with Leu and its incorporation at the TAG codon.
Column 2. Cells grown with 4 mM DMNB-Cys added to the medium produce a 6 fold higher EGFP fluorescence as compared with column 1, consistent with charging of the O-tRNA with DMNB-Cys and incorporation of DMNB-Cys at the TAG codon of the reporter protein during translation.
Column 3. Cells grown with 4 mM DMNB-Sec added to the medium produce similar fluorescence levels of the EGFP protein, compared to column 2, consistent with charging of O-tRNA with DMNB-Sec and incorporation of DMNB-Sec at the TAG codon of the reporter protein during translation.
Column 4. Cells transformed with pGFP-TAG alone produce low levels of functional EGFP and correspond to the TAG nonsense codon suppression errors made by cellular translation system.
Column 5. Fluorescence background in cells lacking EGFP reporter plasmid.
Column 6. Yeast autofluorescence background in untransformed cells.
Column 7. Absence of fluorescence of 4 mM DMNB-Cys solution.
Column 8. Absence of fluorescence of 4 mM DMNB-Sec solution.

These observations are consistent with targeted incorporation of a photocaged Sec residues in the EGFP protein produced in yeast cells.

### Light induced removal of the protecting DMNB group to yield a selenoprotein (Figure 4)

The produced selenoprotein containing a photocaged Sec residue is decaged during a photochemical reaction in which the protective DMNB group is removed from selenium yielding a protein with Sec in place (see Fig. 4). Figure 4 shows the time course of the photolytical decaging of the incorporated DMNB-Sec residue. The 6-His tagged EGFP protein carrying the unnatural amino acid DMNB-Sec at position Tyr39 was purified from yeast cells using standard IMAC procedure. Protein solution (0.2 mg/ml in PBS) was exposed to UV light generated by an Olympus AX70 microscope equipped with excitation filter (340-390 nm band pass). At indicated time points, 20 µl samples were taken for ESI-MS analysis and the EGFP products were quantified based on mass spectrograms. The caging DMNB group was removed in high yield after 40-60 min illumination demonstrating utility of the described approach for selenoprotein production.

### Example 3 Light-controlled reversible protein dimerization

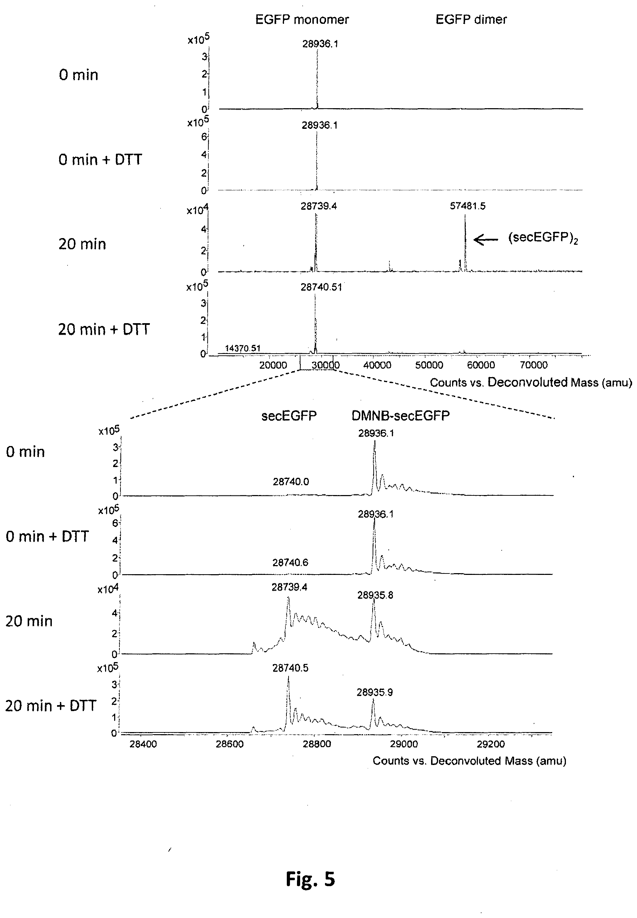

### Chemically reversible dimerization of an engineered sec-protein (Figure 5)

Figure 5 demonstrates the formation of (SecEGFP)₂ dimers upon exposure of the DMNB-SecEGFP protein to UV light for 20 min. It also shows subsequent reduction of the dimer with the reducing agent DTT to yield the SecEGFP protein. At 0 min time point, protein solution consisted of photocaged DMNB-SecEGFP (calculated mass: 28936 Da) irrespective of the addition of the 50 mM DTT. After 20 min exposure of the protein to UV light, a new monomeric SecEGFP (calculated mass: 28741 Da) as well as dimeric (SecEGFP)₂ (calculated mass: 57480 Da) protein products were formed. The protein dimers were completely reduced to yield the monomeric SecEGFP after treatment with 50 mM DTT. Consistent with the MS measurements, the dimerization most likely occurs via the formation of a Se-Se bond between the inserted Sec residues under mildly oxidizing conditions (presence of air oxygen), which is readily reversed in the presence of DTT.

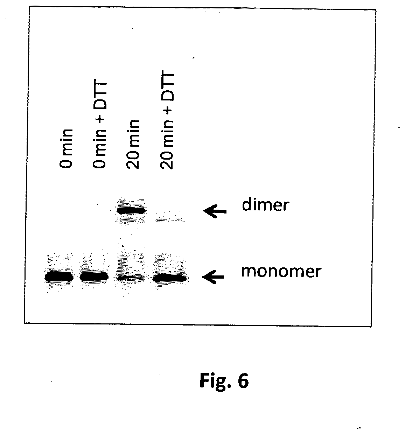

Figure 6. Dimerization of a functionally active selenoprotein.
Aliquots of same samples used in ESI-MS were prepared in standard SDS-PAGE sample buffer (Thermo Fisher) and analyzed by electrophoresis on a 12% polyacrylamide gel. In-gel EGFP fluorescence was visualized using FLA-5100 (Fujifilm, Japan) scanner. Fluorescent (SecEGFP)2 dimers were formed after a 20 min exposure of the DMNB-SecEGFP protein to UV light in high yield, which were then converted to fluorescent monomers by treatment with 50 mM DTT. This experiment illustrates that photolytical decaging, oxidant-promoted dimerization and reductive monomerisation all can occur under mild conditions thereby preserving the functionality (fluorescence) of a protein.

This example shows that further utilisation of the photocaged Sec in peptides or proteins can be useful in cases where light-controlled protein dimerization is desired.

## Claims

1. A method for producing in a translation system a protein comprising an unnatural amino acid at one or more selected positions, the method comprising:
(a) providing a translation system comprising:
(i) an unnatural amino acid that is photocaged selenocysteine;
(ii) an orthogonal aminoacyl-tRNA synthetase (O-RS);
(iii) an orthogonal tRNA (O-tRNA), wherein said O-RS preferentially aminoacylates said O-tRNA with said photocaged selenocysteine; and,
(iv) a nucleic acid encoding said protein, wherein said nucleic acid comprises at least one selector codon that is recognized by said O-tRNA; and,
(b) incorporating said unnatural amino acid at said selected position in said protein during translation of said protein in response to said selector codon, thereby producing said protein comprising said unnatural amino acid at the selected position.

2. A method of claim 1, wherein a photocaged selenocysteine comprises formula I: 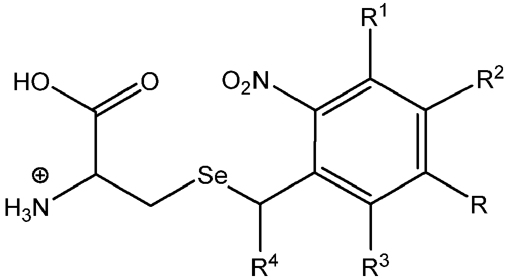 wherein
R is H, alkyl or alkoxy, carboxymethoxy group,
R¹ is H, alkyl, aryl, alkoxy, hydroxy, or a halide group,
R² and R³ each independently represent H, alkyl, aryl, alkoxy, hydroxy group, CO₂R⁵ or halide;
wherein when R and R² or R and R³ is alkoxy, R² or R³ may form a five, six or seven membered cyclic structure in combination with R,
R⁴ is H, methyl, alkyl, CH₂OR⁵ or CO₂R⁵
R⁵ is H or alkyl.

3. A method of claim 2, wherein the photocaged selenocysteine is 4,5-dimethoxy-2-nitrobenzylselenocysteine.

4. The method of claim 1, wherein said translation system comprises a polynucleotide encoding said O-tRNA fused to the SNR52 promoter.

5. The method of claim 1, wherein the selector codon recognized by said O-tRNA is UAG nonsense codon.

6. The method of claim 1, wherein said translation system comprises a nucleic acid that encodes a mutated orthogonal LeuRS.

7. The method of any of preceding claims, wherein said translation system comprises a host cell comprising said unnatural amino acid, said O-RS and said O-tRNA.

8. The method of claim 7, wherein said host cell is a bacterial, yeast or mammalian host cell.

9. The method of claim 1, wherein said method further comprises step (c) removing the photocaging group by exposing translation system or purified protein solution to UV light.

10. The method of claims 1-9 wherein said protein is further used for light-controlled protein dimerization or selective conjugation or targeted labeling or other chemical modification.

11. A photocaged selenocysteine of the formula I: 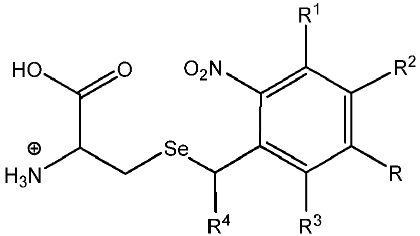 wherein
R is H, alkyl or alkoxy, carboxymethoxy group,
R¹ is H, alkyl, aryl, alkoxy, hydroxy, or a halide group,
R² and R³ each independently represent H, alkyl, aryl, alkoxy, hydroxy group, CO₂R⁵ or halide;
wherein when R and R² or R and R³ is alkoxy, R² or R³ may form a five, six or seven membered cyclic structure in combination with R,
R⁴ is H, methyl, alkyl, CH₂OR⁵ or CO₂R⁵
R⁵ is H or alkyl.

12. A process for producing a selenocysteine of claim 11, which comprises the steps of: reducing of N,N'-blocked selenocystine and reacting with a compound selected from a corresponding substituted (at positions 1,3,4,5 and/or alpha) 2-nitrobenzyl halide, sulfonate, phosphate or carboxylate, followed by de-protection of amino groups.

13. The method according to any of claims 1-10 wherein said one or more selected positions are functionally important Cys or Ser amino acid residues.

14. The method according to any of claims 1-10, wherein one or several photocaged selenocysteine residues are inserted to replace catalytic serine or cysteine residues of a protein and said protein is an enzyme selected from the group of thiol oxidoreductases, protein-tyrosine phosphatases, Cys peptidases and one carbon pyrimidine-5 transferases.

## Patentansprüche

1. Verfahren zur Herstellung eines Proteins in einem Translationssystem, das eine unnatürliche Aminosäure an einer oder mehreren ausgewählten Positionen umfasst, wobei das Verfahren umfasst:
a) Bereitstellen eines Translationssystems, umfassend:
(i) eine unnatürliche Aminosäure, die photocaged Selenocystein ist;
(ii) eine orthogonale Aminoacyl-tRNA-Synthetase (O-RS);
(iii) eine orthogonale tRNA (O-tRNA), wobei die O-RS bevorzugt die O-tRNA mit dem photocaged Selenocystein aminoacyliert; und
(iv) eine für das Protein codierende Nukleinsäure, wobei die Nukleinsäure mindestens ein von der O-tRNA erkanntes Selektorcodon umfasst; und
(b) Einbringen der unnatürlichen Aminosäure an der ausgewählten Position in das Protein während der Translation des Proteins in Reaktion auf das Selektorcodon, wodurch das die unnatürliche Aminosäure an der ausgewählten Position umfassende Protein erzeugt wird.

2. Verfahren nach Anspruch 1, wobei ein photocaged Selenocystein die Formel I umfasst: 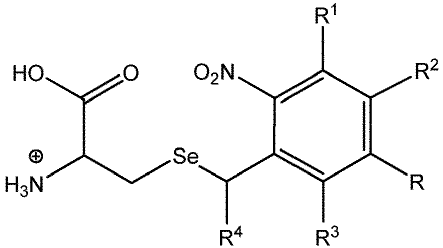 worin
R H, eine Alkyl- oder Alkoxy-, Carboxymethoxygruppe ist,
R¹ H, eine Alkyl-, Aryl-, Alkoxy-, Hydroxy- oder eine Halogenidgruppe ist,
R² und R³ jeweils unabhängig voneinander H, eine Alkyl-, Aryl-, Alkoxy-, Hydroxygruppe, CO₂R⁵ oder ein Halogenid darstellen; wobei, wenn R und R² oder R und R³ Alkoxy sind, R² oder R³ eine fünf-, sechs- oder siebengliedrige cyclische Struktur in Kombination mit R bilden können,
R⁴ H, Methyl, Alkyl, CH₂OR⁵ oder CO₂R⁵ ist,
R⁵ H oder Alkyl ist.

3. Verfahren nach Anspruch 2, wobei das photocaged Selenocystein 4,5-Dimethoxy-2-nitrobenzylselenocystein ist.

4. Verfahren nach Anspruch 1, wobei das Translationssystem ein Polynukleotid umfasst, das für die an den SNR52-Promotor fusionierte O-tRNA codiert.

5. Verfahren nach Anspruch 1, wobei das von der O-tRNA erkannte Selektorcodon ein UAG-Nonsens-Codon ist.

6. Verfahren nach Anspruch 1, wobei das Translationssystem eine Nukleinsäure umfasst, die für eine mutierte orthogonale LeuRS codiert.

7. Verfahren nach einem der vorstehenden Ansprüche, wobei das Translationssystem eine Wirtszelle umfasst, die die unnatürliche Aminosäure, die O-RS und die O-tRNA umfasst.

8. Verfahren nach Anspruch 7, wobei die Wirtszelle eine Bakterien-, Hefe- oder Säugetierwirtszelle ist.

9. Verfahren nach Anspruch 1, wobei das Verfahren weiter den Schritt (c) Entfernen der Photocaging-Gruppe durch Aussetzen des Translationssystems oder der gereinigten Proteinlösung gegenüber UV-Licht umfasst.

10. Verfahren nach Anspruch 1-9, wobei das Protein weiter zur lichtgesteuerten Protein-Dimerisierung oder selektiven Konjugation oder gezielten Markierung oder anderen chemischen Modifizierung verwendet wird.

11. Photocaged Selenocystein der Formel I: 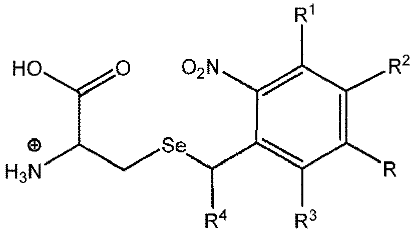 worin
R H, eine Alkyl- oder Alkoxy-, Carboxymethoxygruppe ist,
R¹ H, eine Alkyl-, Aryl-, Alkoxy-, Hydroxy- oder eine Halogenidgruppe ist,
R² und R³ jeweils unabhängig voneinander H, eine Alkyl-, Aryl-, Alkoxy-, Hydroxygruppe, CO₂R⁵ oder ein Halogenid darstellen; wobei, wenn R und R² oder R und R³ Alkoxy sind, R² oder R³ eine fünf-, sechs- oder siebengliedrige cyclische Struktur in Kombination mit R bilden können,
R⁴ H, Methyl, Alkyl, CH₂OR⁵ oder CO₂R⁵ ist,
R⁵ H oder Alkyl ist.

12. Verfahren zur Herstellung eines Selenocysteins nach Anspruch 11, umfassend die Schritte: Reduzieren von N,N'-blockiertem Selenocystin und Umsetzen mit einer Verbindung, ausgewählt aus einem entsprechend substituierten (an den Positionen 1,3,4,5 und/oder alpha) 2-Nitrobenzylhalogenid, -sulfonat, -phosphat oder -carboxylat, gefolgt von einer Schutzgruppenentfernung von Aminogruppen.

13. Verfahren nach einem der Ansprüche 1-10, wobei die eine oder mehreren ausgewählten Positionen funktionell wichtige Cys- oder Ser-Aminosäurereste sind.

14. Verfahren nach einem der Ansprüche 1-10, wobei ein oder mehrere photocaged Selenocysteinreste eingefügt werden, um katalytische Serin- oder Cysteinreste eines Proteins zu ersetzen, und das Protein ein Enzym ist, das aus der Gruppe der Thioloxidoreduktasen, Protein-Tyrosinphosphatasen, Cys-Peptidasen und Ein-Kohlenstoff-Pyrimidin-5-Transferasen ausgewählt ist.

## Revendications

1. Procédé de production dans un système de traduction d'une protéine comprenant un acide aminé non naturel sur une ou plusieurs positions sélectionnées, le procédé comprenant :
(a) la fourniture d'un système de traduction comprenant :
(i) un acide aminé non naturel qui est une sélénocystéine de type photocage ;
(ii) une aminoacyl-ARNt synthétase orthogonale (O-RS) ;
(iii) un ARNt orthogonal (O-ARNt), dans lequel ledit O-RS aminoacylate de préférence ledit O-ARNt avec ladite sélénocystéine de type photocage ; et
(iv) un acide nucléique codant pour ladite protéine, dans lequel ledit acide nucléique comprend au moins un codon sélecteur qui est reconnu par ledit O-ARNt ; et
(b) l'incorporation dudit acide aminé non naturel sur ladite position sélectionnée dans ladite protéine pendant une traduction de ladite protéine en réponse audit codon sélecteur, produisant ainsi ladite protéine comprenant ledit acide aminé non naturel sur la position sélectionnée.

2. Procédé selon la revendication 1, dans lequel une sélénocystéine de type photocage comprend la formule I : 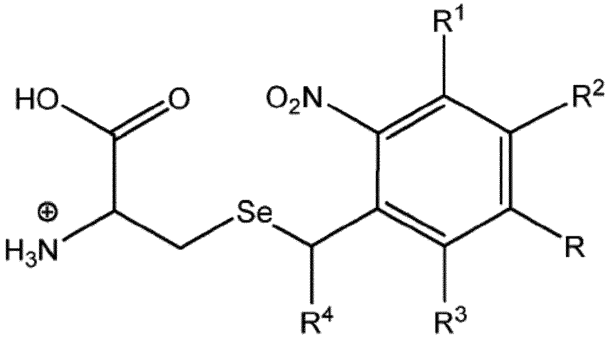 dans laquelle
R est H, un groupe alkyle ou alcoxy, carboxyméthoxy,
R¹ est H, un groupe alkyle, aryle, alcoxy, hydroxy, ou halogénure,
R² et R³ représentent chacun indépendamment H, un groupe alkyle, aryle, alcoxy, hydroxy, CO₂R⁵ ou halogénure ;
dans laquelle lorsque R et R² ou R et R³ sont un groupe alcoxy, R² ou R³ peuvent former une structure cyclique de cinq, six ou sept chaînons en combinaison avec R,
R⁴ est H, un groupe méthyle, alkyle, CH₂OR⁵ ou CO₂R⁵
R⁵ est H ou un groupe alkyle.

3. Procédé selon la revendication 2, dans lequel la sélénocystéine de type photocage est la 4,5-diméthoxy-2-nitrobenzylsélénocystéine.

4. Procédé selon la revendication 1, dans lequel ledit système de traduction comprend un polynucléotide codant pour ledit O-ARNt fusionné au promoteur SNR52.

5. Procédé selon la revendication 1, dans lequel le codon sélecteur reconnu par ledit O-ARNt est le codon non-sens UAG.

6. Procédé selon la revendication 1, dans lequel ledit système de traduction comprend un acide nucléique qui code pour un LeuRS orthogonal muté.

7. Procédé selon l'une quelconque des revendications précédentes, dans lequel ledit système de traduction comprend une cellule hôte comprenant ledit acide aminé non naturel, ledit O-RS et ledit O-ARNt.

8. Procédé selon la revendication 7, dans lequel ladite cellule hôte est une cellule hôte bactérienne, de levure ou de mammifère.

9. Procédé selon la revendication 1, dans lequel ledit procédé comprend en outre l'étape (c) de retrait du groupe formant cage en exposant un système de traduction ou une solution de protéine purifiée à une lumière UV.

10. Procédé selon les revendications 1 à 9, dans lequel ladite protéine est en outre utilisée pour une dimérisation de protéine commandée par la lumière ou une conjugaison sélective ou un marquage ciblé ou une autre modification chimique.

11. Sélénocystéine de type photocage de formule I : 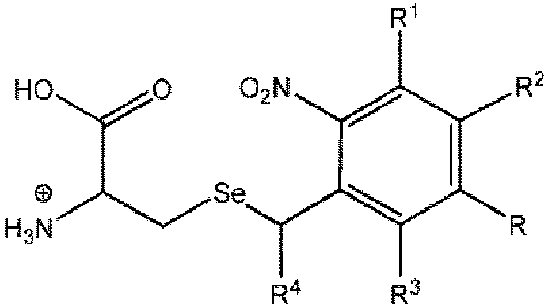 dans laquelle
R est H, un groupe alkyle ou alcoxy, carboxyméthoxy,
R¹ est H, un groupe alkyle, aryle, alcoxy, hydroxy, ou halogénure,
R² et R³ représentent chacun indépendamment H, un groupe alkyle, aryle, alcoxy, hydroxy, CO₂R⁵ ou halogénure ;
dans laquelle lorsque R et R² ou R et R³ sont un groupe alcoxy, R² ou R³ peuvent former une structure cyclique de cinq, six ou sept chaînons en combinaison avec R,
R⁴ est H, un groupe méthyle, alkyle, CH₂OR⁵ ou CO₂R⁵
R⁵ est H ou un groupe alkyle.

12. Procédé de production d'une sélénocystéine selon la revendication 11, qui comprend les étapes de : réduction de la sélénocystéine N,N'-bloquée et réaction avec un composé sélectionné parmi un halogénure 2-nitrobenzyle substitué (sur les positions 1, 3, 4, 5 et/ou alpha), sulfonate ou carboxylate, suivie d'une déprotection de groupes amino.

13. Procédé selon l'une quelconque des revendications 1 à 10, dans lequel lesdites une ou plusieurs positions sélectionnées sont des résidus des acides aminés Cys ou Ser fonctionnellement importants.

14. Procédé selon l'une quelconque des revendications 1 à 10, dans lequel un ou plusieurs résidus de sélénocystéine de type photocage sont insérés pour remplacer des résidus catalytiques de sérine ou de cystéine d'une protéine et ladite protéine est une enzyme sélectionnée dans le groupe des thiol oxydoréductases, protéine-tyrosine phosphatases, Cys peptidases et pyrimidine-5-transférases à un carbone.
